# EUROPEAN PATENT APPLICATION

(11) **EP 4 549 954 A1**
(43) Date of publication of application: **07.05.2025**
(21) Application number: 23830832.4
(22) Date of filing: 08.05.2023
(51) Int. Cl.: G01N 35/08, C12M 1/00, C12M 1/34, G01N 37/00

(54) **CONNECTOR AND CONNECTION STRUCTURE FOR MICROFLUIDIC DEVICE**

(30) Priority: 28.06.2022 JP 2022103764
(71) Applicant: NOK Corporation, Tokyo 105-8585 (JP)
(72) Inventor: ISHIKAWA Terumitsu, Fujisawa-shi, Kanagawa 251-0042 (JP); FUJISAWA Naohiro, Fujisawa-shi, Kanagawa 251-0042 (JP)
(74) Representative: Global IP Europe Patentanwaltskanzlei
(86) International application number: PCT/JP2023/017305
(87) International publication number: WO 2024/004380

(57) **Abstract**

A connector 1 includes a connector body 2 having a tube insertion hole 13 that is formed between a connector upper surface 2a and a connector lower surface 2b and into which a tube can be inserted and connected; a guide part 3 that protrudes downward from the connector lower surface 2b along a lower end peripheral edge of the connector body 2 and is capable of being brought into contact with a part of a micro fluid device; and a lip part 4 that surrounds a lower end opening 13a of the tube insertion hole 13 opened to the connector lower surface 2b, protrudes downward from the connector lower surface 2b, protrudes higher than the guide part 3, and is capable of being brought into contact with a part of the micro fluid device, wherein the connector body 2, the guide part 3, and the lip part 4 are integrally formed by an elastically deformable elastic rubber material.

## Description

### Technical Field

The present invention relates to a connector and a connection structure for a micro fluid device, and more particularly, to a connector interposed between a tube for supplying a fluid to a micro flow path formed inside a micro fluid device and a micro fluid device, and a connection structure for a micro fluid device using the connector.

### Description of the Related Art

Conventionally, there has been known a micro fluid device for forming a fine flow path called a micro flow path having an flow path diameter of several micrometers to several hundred micrometers inside a resinous material or a glass material and performing various operations and processes such as separating, concentrating, reacting, and analyzing while flowing various fluids, including liquids such as a culture solution or a reaction solution, or a powder, through the micro flow path, on a microscale, by applying a semiconductor microfabrication technique or the like.

By using such a micro fluid device, for example, a flow of blood or the like in a blood vessel can be reproduced in a micro flow path, or a biological experiment such as a cell culture or a tissue culture, a medical inspection, or the like can be performed in three dimensions and with a flow of a fluid, compared to the conventional method using a petri dish or the like.

As a result, various experiments and inspections can be performed in a state close to the actual environment and conditions, and further, the use amount of fluids, chemicals, and the like to be used can be reduced by being performed on a microscale, and the reaction and the like can be efficiently performed. As such, there has been an increasing opportunity to use a micro fluid device, particularly in recent years in various fields.

When various operations are performed using a micro fluid device, a supply operation (for example, a liquid supply operation) for stably supplying a fluid such as a culture solution to a fine micro flow path needs to be performed. For example, a method is known in which a micro pipette equipped with a pipette chip is used to inject a predetermined amount of fluid into a micro flow path.

Further, there is also known a method in which conventionally known fluid supply means such as a pump or a syringe are used, and the fluid supply means and the micro fluid device are connected to each other by a resin tube made of a resin material such as silicone rubber, and a certain amount of fluid is continuously supplied into the micro flow path through the tube.

**In** the method of continuously supplying a fluid as described above, it is required to connect the distal end of the tube having a hollow tubular shape and one flow path opening (supply port side) of the micro flow path, and to pour a fluid such as a liquid into the micro flow path at a predetermined flow rate, and to prevent the occurrence of a defect such as the leakage of the fluid from the connecting part of the tube and the flow path opening. Therefore, a connector (resin connector) formed of a resin material or the like may be interposed between the tube and the micro fluid device (for example, refer to Patent Document 1).

As an example of a conventional connector interposed between a tube and a micro fluid device, for example, a connector configured in a so-called "set screw" shape in which a tube insertion hole that passes through the central part and into which a tube can be inserted is provided, and a male screw is formed on one end side of the connector, is known.

On the other hand, a device holder (holder jig) that supports the micro fluid device to which the connector is connected is provided with a connector receiving part in which a female screw that can be screwed with a male screw part of the connector is formed, and the connector receiving part and the flow path opening of the micro flow path of the fluid device are formed in communication.

**In** a state in which a tube made of resin or the like is connected to a tube insertion hole of the connector, when a male screw on the connector side and a female screw on the fluid device side are screwed together, the connector and the fluid device can be connected to each other, and when the connector is fastened and fixed by screwing the male screw and the female screw, the tube and the fluid device can be connected via the connector, and the leakage of fluid from the connecting part of the tube and the fluid device can be prevented by screwing the male screw and the female screw.

As a result, a fluid such as a culture solution supplied from a tube made of resin through a fluid supply unit can be supplied through a supply port to a space inside a micro flow path formed inside the micro fluid device through a connector, and various operations and processes such as culturing of cells and the like can be performed by using fluids supplied into the micro flow path and flowing therethrough.

The fluid that has passed through the micro flow path is discharged from the other flow path opening (discharge port side) of the micro fluid path to the outside of the micro fluid device. In addition, a connector connected to the tube is interposed in the flow path opening on the discharge side in the same way as the supply side, and the fluid is discharged while the leakage of the fluid is prevented between the micro fluid device and the tube.

Furthermore, in the above Patent Document 1, a device structure in which a packing (seal member) formed of a resin material is interposed between the tube and the fluid device to prevent the leakage of the fluid by the seal member, which is a further development of the connection structure of the micro fluid device using the connector that utilizes the screwing of the male screw and the female screw described above, is also proposed.

### Citation List

### Patent Document

[Patent Document 1] JP-A-2006-337032

### SUMMARY OF THE INVENTION

### Problem to be solved by the Invention

However, the connector and the connection structure of the micro fluid device described above may cause the following problems.

In other words, in the case of the connector that utilizes the screwing of the male screw and the female screw, the connector in which the tube is connected needs to be screwed to the connector receiving part, and depending on the degree of fastening of the connector to the connector receiving part (degree of screwing), a part of the tube insertion hole inside the connector may be blocked, or a part of the micro flow path connected to the connector receiving part may be blocked. Therefore, it is necessary to pay sufficient attention to the mounting operation of the connector to the connector receiving part, and mounting such a connector may take time and may impose an excessive work load on the operator, such as troublesome work and complicated adjustment.

Further, there is a possibility that a large amount of time is required for the troublesome work and complicated adjustment, and it may take a lot of time for an operator (a person in charge of an experiment, a work executor, or the like) who performs an experiment such as cell culture to prepare before starting an actual experiment. Further, when an operator is inexperienced in preparing experiments, there may be a variation in the mounting operation (tightening operation) of the connector to the connector receiving part, which can result in unstable flow of the fluid in the micro flow path and make it difficult to perform highly accurate cellular experiments and the like.

On the other hand, even in the case of a connection structure in which a conventional seal member such as a packing is interposed, a defect such as the leakage of the fluid may occur. Further, due to insufficient tightening of the seal member, the seal member may be detached from the device holder, or the seal member may not provide a sufficient seal. As a result, the leakage of the fluid or the like occurs in the same manner as described above, and the experimental accuracy cannot be sufficiently ensured in some cases.

In view of the above circumstances, an object of the present invention is to provide a connector formed with a relatively simple structure for connecting a tube for supplying or discharging fluid to a fluid device to supply a fluid to a micro flow path, and capable of easily performing a connecting operation between a tube and a micro fluid device, and a connection structure for a micro fluid device using the connector.

### Means for Solving the Problems

According to the present invention, a connector and a connection structure for a micro fluid device that solve the above problems are provided below.
[1] A connector comprising: a connector body having a tube insertion hole that is formed between a connector upper surface and a connector lower surface opposite to the connector upper surface and into which a tube used for supplying a fluid to a micro flow path formed inside a micro fluid device or discharging the fluid from the micro flow path can be inserted and connected; a guide part that protrudes downward from the connector lower surface along a lower end peripheral edge of the connector body and is capable of being brought into contact with a part of the micro fluid device; and a lip part that surrounds a lower end opening of the tube insertion hole opened to the connector lower surface, protrudes downward from the connector lower surface, protrudes higher than the guide part, and is capable of being brought into contact with a part of the micro fluid device, wherein the connector body, the guide part, and the lip part are integrally formed of an elastically deformable elastic rubber material.
[2] The connector according to [1], wherein the connector body includes an upper connector body including the connector upper surface and having a cylindrical shape, and a lower connector body including the connector lower surface and having a tapered part extending from a lower end of the upper connector body toward the connector lower surface so as to be enlarged in diameter.
[3] The connector according to [1] or [2], further comprising a flange that protrudes horizontally from a connector-side peripheral surface along an upper end peripheral edge of the connector body.
[4] The connector according to [1] or [2], wherein an insertion hole diameter of the tube insertion hole is set to be smaller than an outer diameter of the tube to be inserted and connected.
[5] The connector according to [3], wherein a flange outer diameter of the flange is set to be larger than a connector mounting hole of a device holder to which the connector can be mounted.
[6] The connector according to [1] or [2], wherein the elastic rubber material is a silicone rubber.
[7] A connection structure for a micro fluid device using the connector according to [1] or [2], comprising: a connector having a connector body having a tube insertion hole; a guide part; and a lip part, wherein the connector body, the guide part and the lip part are integrally formed of an elastically deformable elastic rubber material, and a tube is connected to the tube insertion hole; a micro fluid device having a micro flow path formed therein; and a device holder including an upper holder, a lower holder, and a clamp part that holds the connector and the micro fluid device in a state of being sandwiched between the upper holder and the lower holder, wherein the lower end opening of the tube insertion hole opened to the connector lower surface of the connector is aligned with a position opposed to the flow path opening of the micro flow path of the micro fluid device, and the connector and the micro fluid device are sandwiched between the upper holder and the lower holder from above and below by using the clamp part, thereby holding close contact between a lip part protruding from the connector lower surface of the connector and the micro fluid device.

The connector of the present invention is formed in a relatively simple structure, and the connection between the tube and the micro fluid device can be performed in a simple and short time, and the connection structure for the micro fluid device of the present invention can be constructed. The connection structure for the micro fluid device using the connector of the present invention can reduce the time required for various operations such as culture experiments and preparation for processing, reduce the number of work steps, and eliminate the complexity of the work process.

### BRIEF DESCRIPTION OF THE DRAWINGS

FIG. 1 is a perspective view showing a connector of an embodiment of the present invention.
FIG. 2 is a front view of a connector of the present embodiment.
FIG. 3 is a cross-sectional view showing a connector of the present embodiment.
FIG. 4 is an explanatory view showing a fluid device and a lower holder.
FIG. 5 is an explanatory view showing an upper holder mounted with a connector.
FIG. 6 is an explanatory view showing a state in which the upper holder and the lower holder are superposed on each other.
FIG. 7 is a cross-sectional view schematically showing a state in which the upper holder and the lower holder are superposed on each other in FIG. 6.
FIG. 8 is an enlarged cross-sectional view of a region α enclosed by the broken line in FIG. 7.
FIG. 9 is an explanatory view showing a clamp operation by a clamp part.
FIG. 10 is an explanatory view showing a connection structure of a micro fluid device after the clamp operation.
FIG. 11 is a cross-sectional view schematically showing the connection structure of the micro fluid device of FIG. 10.
FIG. 12 is an enlarged cross-sectional view of a region β enclosed by the broken line in FIG. 11.
FIG. 13 is an explanatory view showing an example of the supply of a fluid to a micro flow path via a plurality of connectors.

### Mode for Carrying out the Invention

Hereinafter, embodiments of a connector and a connection structure for a micro fluid device (hereinafter, simply referred to as a "device connection structure") of the present invention will be described with reference to the drawings. Note that the connector and the device connection structure of the present invention are not limited to those shown below, and various design changes, modifications, improvements, and the like can be made without departing from the gist of the present invention.

### 1. Connector

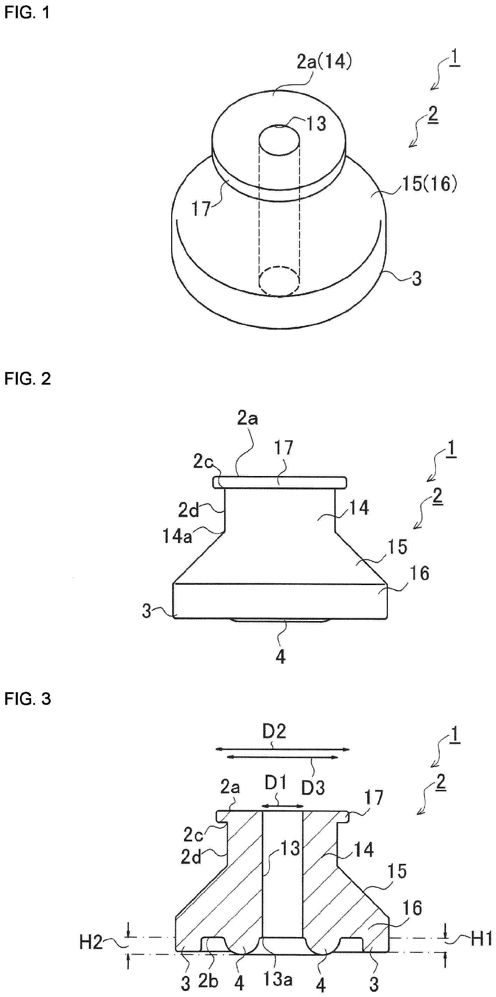

As shown mainly in FIGs. 1 to 3, a connector 1 of an embodiment of the present invention mainly includes a connector body 2, a guide part 3, and a lip part 4, and the connector body 2, the guide part 3, and the lip part 4 are integrally formed of an elastically deformable elastic rubber material.

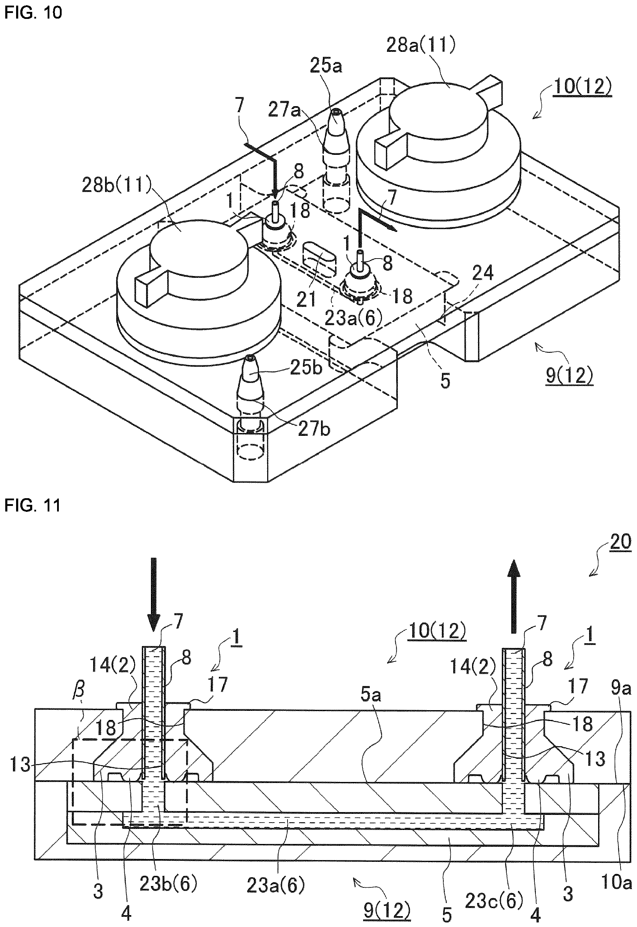

By using the connector 1 of the present embodiment, a tube 8 made mainly of resin for supplying a fluid 7 such as a liquid to a micro fluid path 6 formed inside a micro fluid device 5 (hereinafter, simply referred to as a "fluid device 5") or discharging the fluid 7 from the micro flow path 6 can be connected to the fluid device 5 without leakage of the fluid 7, so that the device connection structure 20 of the present invention can be constructed (see FIGs. 10 to 12).

Here, FIG. 1 is a perspective view showing a schematic configuration of a connector 1 of the present embodiment, FIG. 2 is a front view showing a schematic configuration of a connector 1 of the present embodiment, and FIG. 3 is a cross-sectional view showing a schematic configuration of a connector 1 of the present embodiment.

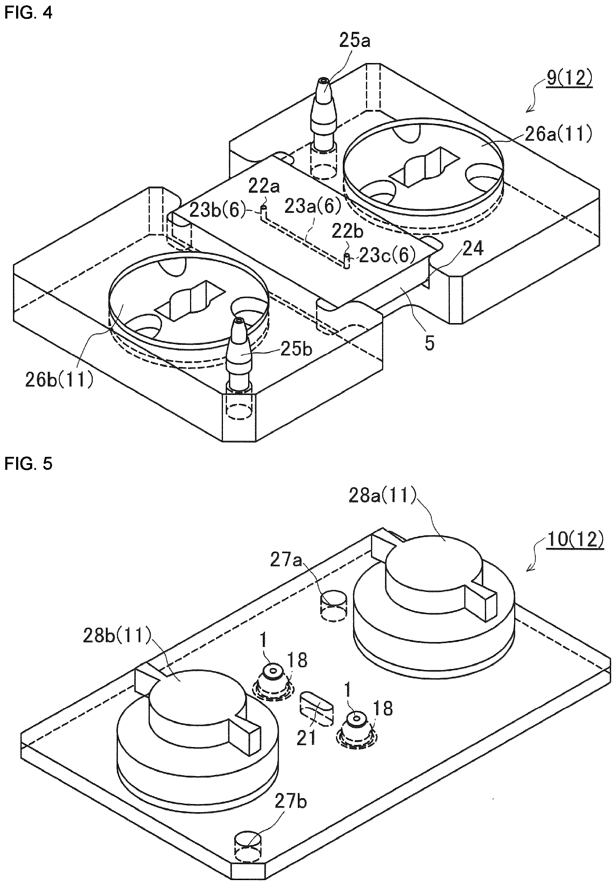

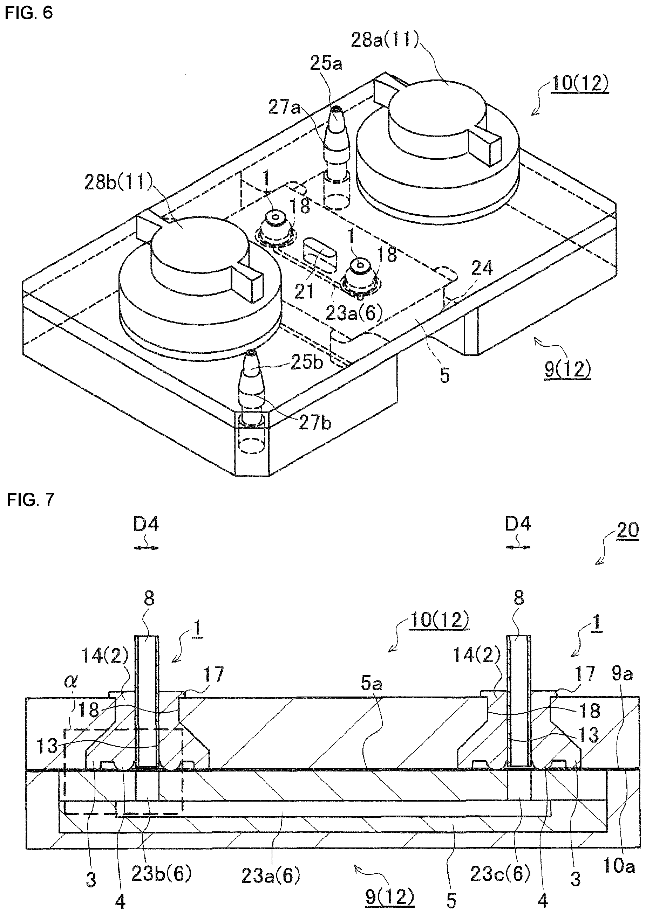

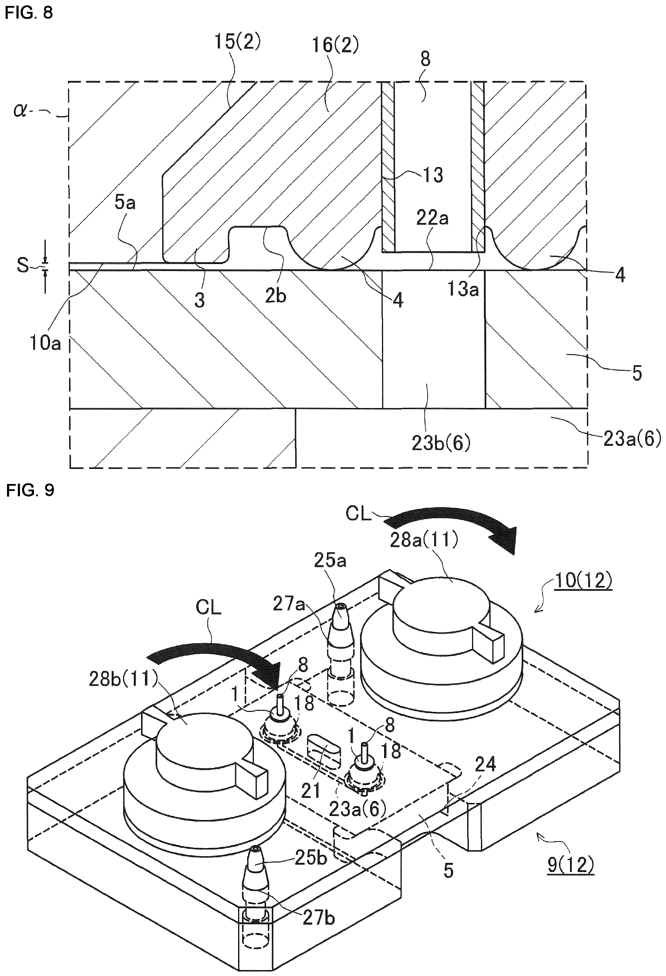

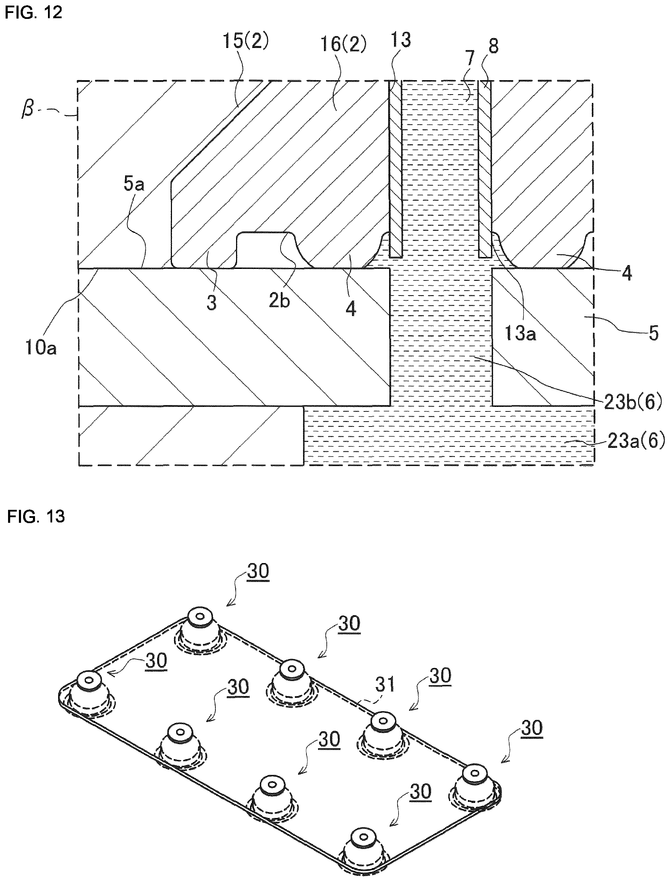

In addition, FIG. 4 is an explanatory view showing a fluid device 5 and a lower holder 9, FIG. 5 is an explanatory view showing an upper holder 10 mounted with a connector 1, FIG. 6 is an explanatory view showing a state in which the upper holder 10 and the lower holder 9 are superposed on each other, FIG. 7 is a cross-sectional view schematically showing a state in which the upper holder 10 and the lower holder 9 are superposed on each other in FIG. 6, FIG. 8 is an enlarged cross-sectional view of a region α enclosed by the broken line in FIG. 7, FIG. 9 is an explanatory view showing a clamp operation by a clamp part 11, FIG. 10 is an explanatory view showing a device connection structure 20 after the clamp operation, FIG. 11 is a cross-sectional view schematically showing the device connection structure 20 of FIG. 10, FIG. 12 is an enlarged cross-sectional view of a region β enclosed by the broken line in FIG. 11, and FIG. 13 is an explanatory view showing an example of the supply of a fluid 7 to a micro flow path 31 via a plurality of connectors 30.

The elastically deformable elastic rubber material for forming the connector 1 of the present embodiment in which the connector body 2, the guide part 3, and the lip part 4 are integrally formed is not particularly limited, and for example, silicon rubber, butylrubber, halogenated butylrubber, vinyl modified butylrubber, ethylene propylene-based rubber, fluoro rubber, acrylic rubber, or hydrogen-added nitrile rubber or the like can be used.

Various operations and processes of the fluid device 5 using the connector 1 in the micro flow path 6 are often performed on a microscale basis, for example in experiments in the biological field such as cell culture, or experiments in the chemical field using various chemicals. Therefore, among the elastic rubber materials listed above, it is preferable to use a silicone rubber that is particularly excellent in biocompatibility and excellent in chemical resistance. The elastic rubber material can be formed into the shape of the connector 1 of the present embodiment by using a molding die made in accordance with the shape of the connector 1 designed in advance and using a well-known rubber molding technique such as extrusion or injection molding.

To explain each component in more detail, a connector body 2 has a tube insertion hole 13 that is formed between a connector upper surface 2a and a connector lower surface 2b opposite to the connector upper surface 2a in parallel thereto. The tube insertion hole 13 is formed so that a tube 8 for supplying a fluid 7 to the micro flow path 6 formed inside the fluid device 5 or discharging the fluid 7 from the micro flow path 6 can be inserted therein for connection (see FIG. 9 or FIG. 10).

As shown in FIG. 3 and the like, the tube insertion hole 13 is formed so as to penetrate the center of the connector of the connector 1 of the present embodiment along the vertical direction and to maintain a constant tube insertion hole diameter D1. Here, the material of the tube 8, which is made of resin and inserted into the tube insertion hole 13, is not particularly limited, and various resin materials and the like can be used as in the above-described connector 1, and it is particularly preferable to use a silicone rubber having excellent biocompatibility and excellent chemical resistance.

Further, as shown mainly in FIGs. 1 to 3, the connector body 2 includes an upper connector body 14 including a connector upper surface 2a, a part of the tube insertion hole 13 formed in the center, and having a cylindrical shape, and a lower connector body 16 including a connector lower surface 2b, a remaining part of the tube insertion hole 13 formed in the center, and including a tapered part 15 extending from a lower end 14a of the upper connector body 14 toward the connector lower surface 2b so as to be enlarged in diameter. That is, the connector body 2 is mainly composed of two members: an upper connector body 14 having a substantially cylindrical shape and a lower connector body 16 having a truncated conical shape.

By providing the connector body 2 with the above-described characteristic configuration, in particular, by configuring the lower connector body to have a tapered part 15 and a substantially trapezoidal cross-section, when the connector body is pressed against the fluid device 5 mounted and supported by a device holder 12 (see FIG. 4 and the like) to be described later, and a compressive load is applied from the vertical direction, the compression excess applied to the tapered part 15 of the connector 1 can be dispersed in the force to press the lip part 4 in the vertical downward direction toward the fluid device 5 (corresponding to the downward direction of the paper surface in FIG. 11), and the force to press the tube 8 inserted into the tube insertion hole 13 toward the center direction of the tube insertion hole 13 (or the tube 8).

This maintains a high sealing property between the lip part 4 and the device surface 5a of the fluid device 5, and makes it possible to strengthen the adhesion between the connector 1 and the tube 8. As a result, it is possible to suppress occurrence of a defect such as leakage of the fluid 7 between the connector 1 and the fluid device 5 and between the connector 1 and the tube 8.

Further, the guide part 3 of the connector 1 of the present embodiment protrudes downward from the connector lower surface 2b along a lower end peripheral edge of the connector body 2 (lower connector body 16), and is formed so as to be able to be brought into contact with a part of the fluid device 5. Since the connector 1 includes the guide part 3, the connector 1 capable of being brought into contact with a part of the fluid device 5, is brought into contact with the fluid device 5 with a gap between the device surface 5a of the fluid device 5 and the connector lower surface 2b. That is, the connector lower surface 2b can be positioned above the device surface 5a by the guide part protruding height H1 of the guide part 3 protruding from the connector lower surface 2b without the connector lower surface 2b directly contacting the device surface.

In addition, the lip part 4 of the connector 1 of the present embodiment is formed to surround the periphery of a lower end opening 13a of the tube insertion hole 13 opened to the connector lower surface 2b in a circular shape, to protrude downward from the connector lower surface 2b and protrude higher than the guide part protruding height H1 of the guide part 3, and to be capable of being brought into contact with a part of the fluid device 5. That is, the lip part 4 has a lip part protruding height H2 higher than the guide part protruding height H1 of the guide part 3, and for example, as shown in a lateral view of the connector 1 shown in FIG. 2 or a cross-sectional view of the connector 1 shown in FIG. 3, the tip of the lip part 4 is formed so as to protrude beyond the tip of the guide part 3.

Therefore, when no compressive load is applied to the connector 1, the tip of the lip part 4 protrudes beyond the guide part 3 as described above. On the other hand, when a compressive load is applied from the vertical direction to the connector 1, the lip part 4 formed integrally with the connector body 2 or the like of an elastically deformable elastic rubber material is deformed such that the tip part of the lip part 4 is crushed, and the guide part protruding height H1 of the guide part 3 and the lip part protruding height H2 of the lip part 4 temporarily become the same height. As a result, the elastic repulsive force of the lip part 4 exerts a strong pressing force on the device surface 5a that comes into contact with the lip part. As a result, a high sealing property can be achieved between the lip part 4 and the fluid device 5.

In addition to the above-described configuration, the connector 1 of the present embodiment further includes a flange 17 protruding horizontally from a connector-side peripheral surface 2d along an upper end peripheral edge 2c of the connector body 2 (the upper connector body 14). It is preferable that the first connector diameter D2 of the upper connector body 14 including the flange 17 (corresponding to the flange outer diameter in the present invention) is formed so as to be approximately 0.3mm to 0.5mm larger than the second connector diameter D3 of the upper connector body 14 not including the flange 17 (the connector-side peripheral surface 2d corresponds to the outer surface) (see FIG. 3).

By providing the flange 17 in this manner, the flange 17 corresponds to a so-called "barb portion", and when the connector 1 is mounted to a connector mounting hole 18 of the upper holder 10, the movement of the connector 1 can be restricted. As a result, it is possible to provide a fall-off prevention function that prevents the connector 1, once mounted, from easily falling off the upper holder 10.

On the other hand, the second connector diameter D3 not including the flange 17 is formed so as to substantially coincide with the hole diameter of the narrowest part of the connector mounting hole 18. As described above, the flange 17 is set such that the first connector diameter D2 (=corresponding to the flange outer diameter) is larger than the hole diameter of the narrowest portion of the connector mounting hole 18.

In the connector 1 of the present embodiment, the tube insertion hole diameter D1 (see FIG. 3) of the tube insertion hole 13 formed through the connector body 2 (the upper connector body 14 and the lower connector body 16) is set to be smaller than the tube outer diameter D4 (see FIG. 7) of the tube 8 inserted into and connected to the tube insertion hole 13.

Since the connector 1 is made of an elastically deformable elastic rubber material, the tube 8 can be inserted into the tube insertion hole 13 as it is even when the tube insertion hole diameter D1 is set smaller than the tube outer diameter D4 of the tube 8 as described above, and the outer surface of the tube 8 is brought into close contact with the tube insertion hole 13. As a result, the tube 8 and the connector 1 can be connected with a simple operation, and the leakage of the fluid 7 or the like from between the tube 8 and the tube insertion hole 13 is prevented. The tube insertion hole diameter D1 and the tube outer diameter D4 can be set arbitrarily, but
in consideration of the mounting operation and the like, for example, it is preferable to set the tube insertion hole diameter D1 to be approximately 0.05 to 0.10mm smaller than the tube outer diameter D4.

### 2. Device connection structure

Next, the connection structure 20 of the fluid device using the connector 1 of the present embodiment described in 1. above (corresponding to the connection structure of the micro fluid device of the present invention. Hereinafter, simply referred to as the "device connection structure 20") will be described. Note that, in the device connection structure 20 of the present embodiment, the configurations of the connector 1 and the like described above are denoted by the same reference numerals, and detailed descriptions thereof will be omitted.

The device connection structure 20 of the present embodiment mainly includes a connector 1 made of an elastic rubber material such as silicone resin, a fluid device 5 formed with a micro flow path 6 therein, and a device holder holding the connector 1 and the fluid device 5 sandwiched therebetween.

As shown in FIGs. 4 to 12, the device holder 12 mainly includes an upper holder 10 having a connector mounting hole 18 into which the connector 1 can be mounted and being disposed from above the fluid device 5, a lower holder 9 for mounting the fluid device 5 and supporting it from below, and a clamp part 11 for fixing the connector 1 mounted on the upper holder 10 and the fluid device 5 mounted and supported on the lower holder 9 in close contact with each other in a state in which the upper holder 10 and the lower holder 9 are vertically overlapped.

By providing the above three configurations, it is possible to construct a device connection structure 20 in which the lower end opening 13a of the tube insertion hole 13 of the connector 1 is aligned to a position opposed to the flow path openings 22a and 22b of the micro flow path 6 of the fluid device 5, the connector 1 and the fluid device 5 are brought into close contact by using the clamp part 11, and the state is maintained (see FIGs. 8 and 11).

Details of the device connection structure 20 of the present embodiment and details of the functions and effects of the connector 1 by the device connection structure 20 will be described below mainly with reference to FIGs. 4 to 10.

First, a fluid device 5 with a micro flow path 6 is placed in a lower holder 9 (see FIG. 4). Here, the fluid device 5 has a substantially rectangular parallelepiped shape, and the micro flow path 6 is formed inside the fluid device 5. Further, the micro flow path 6 is formed with a straight pipe section 23a having a straight pipe-shape formed along the longitudinal direction of the fluid device 5, and a pair of vertical pipe sections 23b and 23c that are bent vertically upward (toward the device surface 5a) from the respective ends of the straight pipe section 23a and have flow path openings 22a and 22b that open to the device surface 5a.

The micro flow path 6 having such a configuration allows the fluid 7 supplied from one flow path opening 22a (corresponding to the supply port) that comes into contact with the connector 1 connected to the tube 8 to pass through one of the vertical pipe section 23b, the straight pipe section 23a, and the other vertical pipe section 23c (corresponding to the discharge port), and to be discharged to the outside of the fluid device 5 and the device connection structure 20 through the other flow path opening 22b.

Here, the lower holder 9 is configured to include a device mounting and supporting part 24 for mounting the fluid device 5 and supporting it from below, positioning projections 25a and 25b for aligning the overlapping position with the upper holder 10, and clamp receiving parts 26a and 26b constituting a part of the clamp part 11 for performing a clamp operation of the upper holder 10 and the lower holder 9, respectively. Furthermore, the device mounting and supporting part 24 is formed such that the device surface 5a of the mounted fluid device 5 and the holder upper surface 9a of the lower holder 9 are flush with each other (see FIGs. 7 and 8).

On the other hand, as shown in FIG. 5, the upper holder 10 has a connector mounting hole 18 formed therethrough, into which the connector 1 can be mounted, and further includes positioning holes 27a and 27b provided so as to correspond to the positions of the positioning projections 25a and 25b of the lower holder 9, clamp operation parts 28a and 28b constituting a part of the clamp part 11 for performing a clamp operation, and an observation hole 21 for observing a state in the micro flow path 6.

Here, in the upper holder 10, a pair of connector mounting holes 18 are provided in parallel, and a position where the connector mounting holes 18 are provided is a position opposed to flow path openings 22a and 22b of the above-described micro flow path 6 when the upper holder 10 and the lower holder 9 are overlapped. That is, the lower end opening 13a of the tube insertion hole 13 of the connector 1 mounted in the connector mounting hole 18 and flow path openings 22a and 22b of the micro flow path 6 are opposed to each other.

As described above, a flange 17 is provided at the upper part of the connector 1 (see FIG. 3 and the like). The first connector diameter D2 of the connector body 2 (the upper connector body 14) including the flange 17 is formed to be larger than the hole diameter (=the second connector diameter D3) of the connector mounting hole 18.

Therefore, when the connector 1 is mounted in the connector mounting hole 18 of the upper holder 10, the connector 1, which is formed of an elastically deformable elastic rubber material (for example, silicone) and is in the vicinity of the flange 17, is crushed and deformed so as to be smaller than the hole diameter of the connector mounting hole 18, and then the connector is mounted. After the upper part of the connector body 2 including the flange 17 passes through the connector mounting hole 18, the force of crushing the connector 1 is released.

As a result, the connector body 2 in the vicinity of the deformed flange 17 is elastically returned, and the connector 1 is fitted into the connector mounting hole 18 of the upper holder 10 as shown in FIG. 7, for example. Further, the connector mounting hole 18 provided in the upper holder 10 is provided with a hole shape so as to substantially match the appearance shape of the connector 1 having a tapered part 15, and the height of the tip of the guide part 3 and the holder lower surface 10a of the upper holder 10 are set to coincide with each other when the connector 1 is fitted into the connector mounting hole 18.

Further, the movement in the vertical direction in FIG. 7 of the connector 1 in a state of being mounted on the upper holder 10 is restricted by the action of the flange 17 provided in the connector 1. Consequently, when the fluid 7 is supplied to the micro flow path 6 or when the fluid 7 is discharged from the micro flow path 6, the connector 1 is not easily detached from the upper holder 10. That is, the flange 17 can function as a so-called "barb portion".

Next, as shown in FIG. 6, the upper holder 10 to which a pair of connectors 1 are mounted is superimposed on the lower holder 9 to which the fluid device 5 is mounted and supported. At this time, a pair of positioning projections 25a and 25b provided in the lower holder 9 are respectively inserted into a pair of positioning holes 27a and 27b provided in the upper holder 10. As a result, the upper holder 10 and the lower holder 9 can be overlapped with each other at the correct position.

At this time, the clamp operation by the clamp part 11 is not performed. Therefore, as shown in FIG. 8 in which FIG.7 and a partial area of FIG. 7 are enlarged, a gap S is formed between the upper holder 10 and the lower holder 9. That is, since the lip part 4 protrudes from the connector lower surface 2b higher than the guide part protruding height H1 of the guide part 3, the lip part 4 is in contact with the device surface 5a of the fluid device 5. Here, FIG. 8 is an enlarged view of a region α surrounded by a broken line in FIG. 7. FIG. 7 shows a state in which the tube 8 is inserted into the tube insertion hole 13. Further, the tube 8 is shown only in the vicinity of the connector 1, and other parts are not shown (the same in FIGs. 9 to 11).

Thereafter, as shown in FIG. 9, a clamp operation is performed in which the clamp operation parts 28a and 28b of the clamp part 11 are rotated clockwise (rotated in the clamp operation direction CL) while the tubes 8 made of resin are inserted into the tube insertion holes 13 of the pair of connectors 1. By the clamp operation, a force is applied in a direction that brings the upper holder 10 and the lower holder 9 close to each other, and the holder lower surface 10a of the upper holder 10 and the holder upper surface 9a of the lower holder 9 come into contact with each other. At this time, a compressive load is applied to the connector 1 mounted on the upper holder 10 and the fluid device 5 mounted and supported on the lower holder 9 in the vertical direction so as to bring them close to each other.

That is, a force that eliminates the above-described gap S between the holder lower surface 10a and the holder upper surface 9a is applied. The connector 1 is integrally formed of silicone rubber, which is an elastic rubber material, and when such a compressive load is applied, a part of the lip part 4 (particularly a tip part) provided in the connector 1 is deformed by contact with the device surface 5a (see FIGs. 10 to 12). Consequently, the above-described gap S is eliminated, and the gap between the lower end opening 13a of the tube insertion hole 13 of the connector 1 and the flow path opening 22a (or flow path opening 22b) of the micro flow path 6 is sealed by the lip part 4 formed so as to surround the lower end opening 13a. Further, as described above, since the connector 1 includes the lower connector body 16 having the tapered part 15, the connection state between the tube 8 inserted into the tube insertion hole 13 and the connector 1 is stabilized. As a result, it is possible to regulate the movement of the fluid 7 supplied through the tube 8, from the sealed space to the lateral direction of FIG. 7 and FIG. 8. Therefore, the construction of the device connection structure 20 of the present embodiment is completed.

While maintaining the sealed state between the tube 8 and the connector 1 and between the connector 1 and the fluid device 5 in this manner, the fluid 7 is supplied to the micro flow path 6 through one connector 1 connected to the tube 8, as shown in FIGs. 10 to 12. Thereby, the fluid 7 can be stably supplied to the micro flow path 6 while a sufficient sealing property between the lip part 4 and the device surface 5a is maintained by the lip part 4 and a sealing property between the tube 8 and the connector 1 is maintained, and operations and processes for various experiments such as culturing of cells and the like can be performed.

**In** particular, since the connector 1 of the present embodiment has the lower connector body 16 having the tapered part 15, the close state between the tube 8 and the tube insertion hole 13 of the connector 1 and the close state between the lip part 4 of the connector 1 and the device surface 5a of the fluid device 5 can be strengthened when a compressive load in the vertical direction is applied to the connector 1 and the fluid device 5 by the clamp operation. This prevents leakage of the fluid 7 at the connecting part of the tube 8 and the fluid device 5. Further, since the lip part 4 protrudes beyond the guide part 3, the elastic deformation force of the lip part 4 can be increased, and the sealing property of the fluid 7 can be further improved. Further, the connector mounting hole 18 of the upper holder 10 of the device holder 12 is formed with a tapered part corresponding to the tapered part 15 of the connector 1. This makes it possible to strengthen the close contact between the tube 8 and the tube insertion hole 13 of the connector 1 and the close contact between the lip part 4 of the connector 1 and the device surface 5a of the fluid device 5, and the upper holder 10 and the connector 1 can be brought into close contact with each other, thereby improving the sealing property between the connector 1 and the device holder 12.

**In** the connector 1 and the device connection structure 20 of the present embodiment described above, one connector 1 is connected to each of the flow path openings 22a, 22b, and the like of the micro flow path 6, but the present invention is not limited thereto, and, for example, as shown in FIG. 13, as long as it is possible to apply a sufficiently compressive load by a device holder (not shown), two or more connectors 30 may be provided to match the flow path openings of the fluid device side.

### Industrial Applicability

The connector and the fluid connection structure of the present invention can be suitably used in various processes and operations in a micro flow path by a micro fluid device.

### Description of Reference numerals

1, 30: connector
2: connector body
2a: connector upper surface
2b: connector lower surface
2c: upper end peripheral edge
2d: connector-side peripheral surface
3: guide part
4: lip part
5: fluid device (micro fluid device)
5a: device surface
6, 31: micro flow path
7: fluid
8: tube
9: lower holder
9a: holder upper surface
10: upper holder
10a: holder lower surface
11: clamp part
12: device holder
13: tube insertion hole
13a: lower end opening
14: upper connector body
14a: lower end
15: tapered part
16: lower connector body
17: flange
18: connector mounting hole
20: device connection structure (connection structure of micro fluid device)
21: observation hole
22a, 22b: flow path opening
23a: straight pipe section
23b, 23c: vertical pipe section
24: device mounting and supporting part
25a, 25b: positioning projection
26a, 26b: clamp receiving part
27a, 27b: positioning hole
28a, 28b: clamp operation part
CL: clamp operation direction
D1: tube insertion hole diameter
D2: first connector diameter
D3: second connector diameter
D4: tube outer diameter
H1: guide part protruding height
H2: lip part protruding height
S: gap
α, β: region enclosed by the broken line

## Claims

1. A connector comprising:
a connector body having a tube insertion hole that is formed between a connector upper surface and a connector lower surface opposite to the connector upper surface and into which a tube used for supplying a fluid to a micro flow path formed inside a micro fluid device or discharging the fluid from the micro flow path can be inserted and connected;
a guide part that protrudes downward from the connector lower surface along a lower end peripheral edge of the connector body and is capable of being brought into contact with a part of the micro fluid device; and
a lip part that surrounds a lower end opening of the tube insertion hole opened to the connector lower surface, protrudes downward from the connector lower surface, protrudes higher than the guide part, and is capable of being brought into contact with a part of the micro fluid device,
wherein the connector body, the guide part, and the lip part are integrally formed of an elastically deformable elastic rubber material.

2. The connector according to claim 1, wherein the connector body includes
an upper connector body including the connector upper surface and having a cylindrical shape, and
a lower connector body including the connector lower surface and having a tapered part extending from a lower end of the upper connector body toward the connector lower surface so as to be enlarged in diameter.

3. The connector according to claim 1 or 2, further comprising a flange that protrudes horizontally from a connector-side peripheral surface along an upper end peripheral edge of the connector body.

4. The connector according to claim 1 or 2, wherein an insertion hole diameter of the tube insertion hole is set to be smaller than an outer diameter of the tube to be inserted and connected.

5. The connector according to claim 3, wherein a flange outer diameter of the flange is set to be larger than a connector mounting hole of a device holder to which the connector can be mounted.

6. The connector according to claim 1 or 2, wherein the elastic rubber material is a silicone rubber.

7. A connection structure for a micro fluid device using the connector according to claim 1 or 2, comprising:
a connector having a connector body having a tube insertion hole; a guide part; and a lip part, wherein the connector body, the guide part and the lip part are integrally formed of an elastically deformable elastic rubber material, and a tube is connected to the tube insertion hole;
a micro fluid device having a micro flow path formed therein; and
a device holder including an upper holder, a lower holder, and a clamp part that holds the connector and the micro fluid device in a state of being sandwiched between the upper holder and the lower holder,
wherein the lower end opening of the tube insertion hole opened to the connector lower surface of the connector is aligned with a position opposed to the flow path opening of the micro flow path of the micro fluid device, and the connector and the micro fluid device are sandwiched between the upper holder and the lower holder from above and below by using the clamp part, thereby holding close contact between a lip part protruding from the connector lower surface of the connector and the micro fluid device.
